# EUROPEAN PATENT SPECIFICATION

(11) **EP 2 313 097 B1**
(45) Date of publication and mention of the grant of the patent: **27.03.2013**
(21) Application number: 09780399.3
(22) Date of filing: 09.07.2009
(51) Int. Cl.: A61K 31/453, A61K 31/496, A61K 31/506, A61K 38/05, A61K 39/395, A61K 45/06, A61P 35/00

(54) **THERAPEUTIC COMBINATION COMPRISING AN AURORA KINASE INHIBITOR AND ANTIPROLIFERATIVE AGENTS**
THERAPEUTISCHE KOMBINATION MIT EINEM AURORA-KINASE-INHIBITOR UND ANTIPROLIFERATIVEN MITTELN
COMBINAISON THÉRAPEUTIQUE RENFERMANT UN INHIBITEUR DE LA KINASE AURORA ET DES AGENTS ANTIPROLIFÉRATIFS

(30) Priority: 24.07.2008 US 83236 P
(43) Date of publication of application: 27.04.2011
(73) Proprietor: Nerviano Medical Sciences S.r.l., 20014 Nerviano (MI) (IT)
(72) Inventor: PESENTI, Enrico, I-20015 Parabiago (IT); BALLINARI, Dario, I-20097 San Donato Milanese (IT); MOLL, Juergen, I-22070 Appiano Gentile (IT)
(86) International application number: PCT/EP2009/058779
(87) International publication number: WO 2010/009985

(56) References cited:
- WO-A1-2005/005427
- WO-A1-2006/046735
- WO-A1-2007/059299
- WO-A1-2007/132215
- WO-A1-2008/026768
- WO-A2-2007/113212
- WO-A2-2008/057512
- US-A1- 2008 131 526
- PAQUETTE RONALD L ET AL: "PHA-739358, an aurora kinase inhibitor, induces clinical responses in chronic myeloid leukemia harboring T315I mutations of BCR-ABL" November 2007 (2007-11), BLOOD, VOL. 110, NR. 11, PART 1, PAGE(S) 312A , 49TH ANNUAL MEETING OF THE AMERICAN-SOCIETY-OF-HEMATOLOGY; ATLANTA, GA, USA; DECEMBER 08 -11, 2007 , XP008118717 ISSN: 0006-4971 paragraph [0001]

## Description

### FIELD OF THE INVENTION

The present disclosure relates in general to the field of cancer treatment and, more particularly, discloses an anti-tumor composition comprising an aurora kinase inhibitor and an antibody inhibiting a growth factor and/or a proteasome inhibitor and/or a kinase inhibitor having a synergistic antiproliferative effect.

### BACKGROUND OF THE INVENTION

The malfunctioning of protein kinases (PKs) is the hallmark of numerous diseases. A large share of the oncogenes and proto-oncogenes involved in human cancers code for PKs. The enhanced activities of PKs are also implicated in many non-malignant diseases, such as benign prostate hyperplasia, familial adenomatosis, polyposis, neuro-fibromatosis, psoriasis, vascular smooth cell proliferation associated with atherosclerosis, pulmonary fibrosis, arthritis, glomerulonephritis and post-surgical stenosis and restenosis.

PKs are also implicated in inflammatory conditions and in the multiplication of viruses and parasites. PKs may also play a major role in the pathogenesis and development of neurodegenerative disorders.

For a general reference to PKs malfunctioning or disregulation see, for instance, Current Opinion in Chemical Biology, 1999, 3, 459-465.

Among the several protein kinases known in the art as being implicated in the growth of cancer cells are Aurora kinases, in particular Aurora-2.

Aurora-2 was found to be over-expressed in a number of different tumour types. Its gene locus maps at 20q13, a chromosomal region frequently amplified in many cancers, including breast [Cancer Res. 1999, 59(9), 2041-4] and colon cancers.

20q13 amplification correlates with poor prognosis in patients with node-negative breast cancer and increased Aurora-2 expression is indicative of poor prognosis and decreased survival time in bladder cancer patients [J. Natl. Cancer Inst., 2002, 94(17), 1320-9]. For a general reference to the Aurora-2 role in the abnormal centrosome function in cancer see also Molecular Cancer Therapeutics, 2003, 2, 589-595.

Several heterocyclic compounds are known in the art as protein kinase inhibitors. Among them, 3-carboxamido-pyrazoles and 3-ureido-pyrazoles and derivatives thereof, have been disclosed as protein kinase inhibitors in the international patent applications WO01/12189, WO12188, WO02/48114 and WO02/70515.

Fused bicyclic compounds comprising pyrazole moieties and possessing kinase inhibitory activity have been also disclosed in WO00/69846, WO02/12242, WO03/028720 and WO03/97610.

Specific tetrahydropyrrolo[3,4-c]pyrazole derivatives have been revealed to be potent ATP-competitive inhibitors of Aurora kinases (Fancelli, D., et al.: Journal of Medicinal Chemistry. 2005, vol.48, no.8, p.3080-3084. PCT/WO 2005005427). Surprisingly, it has been found that the antineoplastic effect, i.e. especially the delay of progression or treatment of a proliferative disease, in particular the treatment of a tumour that is refractory to other chemotherapeutics known as antineoplastic agents, of the tetrahydropyrrolo[3,4-c]pyrazole derivatives above is greatly enhanced when they are administered in combination with certain antineoplastic agents. In particular, the antineoplastic effect of such combination is greater than the effects that can be achieved with either type of combination partner alone, i.e. greater than the effects of a monotherapy using only one of the combination partners.

The present invention thus discloses new combinations of a tetrahydropyrrolo[3,4-c]pyrazole derivative with known pharmaceutical agents that are particularly suitable for the treatment of proliferative disorders, especially cancer. More specifically, the combinations of the present invention are very useful in therapy as antitumor agents and lack, in terms of both toxicity and side effects, the drawbacks associated with currently available antitumor drugs.

### SUMMARY OF THE INVENTION

The present invention provides a therapeutic combination comprising (a) Compound 1 of formula (A): 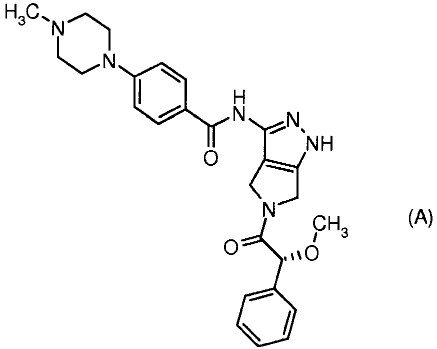 and (b) bortezomib, wherein the active ingredients are present in each case in free form or in the form of a pharmaceutically acceptable salt or any hydrate thereof.

The present invention also provides a combined preparation for simultaneous, separate or sequential use of the combination as described above.

The present invention also provides a pharmaceutical composition comprising a combination as described above which is further admixed with a pharmaceutically acceptable carrier, diluent or excipient.

The present invention further discloses a method of treating proliferative disorders by administering to a patient in need thereof a therapeutically effective amount of the combination as described above.

### DETAILED DESCRIPTION OF THE INVENTION

The present invention provides, in a first embodiment, a therapeutic combination comprising (a) Compound 1 of formula (A): 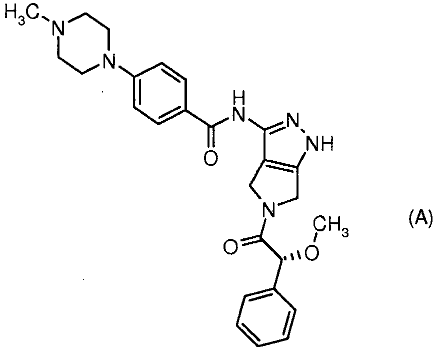 and (b) bortezomib, wherein the active ingredients are present in each case in free form or in the form of a pharmaceutically acceptable salt or any hydrate thereof.

In another embodiment the combination according to the invention is a combined preparation for simultaneous, separate or sequential use.

A further embodiment relates to the combination according to the invention in for use treating or delaying the progression of a proliferative disorder, wherein the use comprises the simultaneous, sequential or separate administration to a patient in need thereof of the therapeutic combination.

In a still further embodiment the invention provides a pharmaceutical composition comprising a combination according to the invention admixed with a pharmaceutically acceptable carrier, diluent or excipient.

Compound 1 of formula (A) has the chemical name N-{5-[(2R)-2-methoxy-2-phenylethanoyl]-1,4,5,6-tetrahydropyrrolo[3,4-c] pyrazol-3-yl}-4-(4-methylpiperazin-1-yl)benzamide. It can be prepared as described in WO 2005/005427, is endowed with protein kinase inhibitory activity and is thus useful in therapy as antitumor agent.

Pharmaceutically acceptable salts of the compound of formula (A) include the acid addition salts with inorganic or organic acids, e.g., nitric, hydrochloric, hydrobromic, sulphuric, perchloric, phosphoric, acetic, trifluoroacetic, propionic, glycolic, lactic, oxalic, malonic, malic, maleic, mesylate, tartaric, citric, benzoic, cinnamic, mandelic, methanesulphonic, isethionic and salicylic acid and the like.

Bortezomib, marketed as Velcade® by Millennium Pharmaceuticals, was the first therapeutic proteasome inhibitor to be tested in humans. It is approved in the U.S. for treating relapsed multiple myeloma and mantle cell lymphoma.

In the present invention, each of the active ingredients of the combination is provided in an amount effective to produce a synergistic antineoplastic effect.

The present invention also discloses a combination for use in lowering the side effects caused by antineoplastic therapy with an antineoplastic agent in mammals, including humans, in need thereof, the use comprising administering to said mammal a combined preparation comprising the compound of formula (A) as defined above and bortezomib, in amounts effective to produce a synergistic antineoplastic effect.

By the term "a synergistic antineoplastic effect" as used herein is meant the inhibition of the growth of the tumor, preferably the complete regression of the tumor, by administering an effective amount of the combination of compound 1 of formula (A) as defined above and bortezomib to mammals, including humans.

The term "combined preparation" as used herein defines especially a "kit of parts"in the sense that the combination components (a) and (b) as defined above can be dosed independently or by use of different fixed combinations with distinguished amounts of the combination components (a) and (b), i.e. simultaneously or at different time points. The elements of the kit of parts can then, e.g., be administered simultaneously or chronologically staggered, that is at different time points and with equal or different time intervals for any part of the kit of parts. Most preferably, the time intervals are chosen such that the effect on the treated disease in the combined use of the parts is greater than the effect which would be obtained by use of only any one of the combination components (a) and (b). The ratio of the total amounts of the combination component (a) to the combination component (b) to be administered in the combined preparation can be varied, e.g. in order to cope with the needs of a patient sub-population to be treated or the needs of the single patient which different needs can be due to the particular disease, age, sex, body weight, etc. of the patients. Preferably, there is at least one beneficial effect, e.g., a mutual enhancing of the effect of the combination components (a) and (b), in particular a synergism, e.g. a more than additive effect, additional advantageous effects, less side effects, less toxicity, or a combined therapeutic effect in a non-effective dosage of one or both of the combination components (a) and (b), and very preferably a strong synergism of the combination components (a) and (b).

By the term "administered" or "administering" as used herein is meant parenteral and /or oral administration. By "parenteral" is meant intravenous, subcutaneous and intramuscular administration.

In the method of the subject invention, for the administration of compound 1 of formula (A), the course of therapy generally employed is in the range from 100 mg/m²/day to 1500 mg/m²/day of body surface area for up to 21 consecutive days. More preferably, the course therapy employed is from about 150 mg/m²/day to about 350 mg/m²/day of body surface area for up to 21 consecutive days.

In a particularly preferred regimen, the compound of formula (A) is administered in a dose of 190 or 250 mg/m²lday of body surface area for three hours infusion on day 1 and 8 of a three weeks cycle. Other possible therapeutic schedules are disclosed, for example, in WO2008/052931 published May 8, 2008.

Compound 1 of formula (A) can be administered in a variety of dosage forms, e.g., orally, in the form of tablets, capsules, sugar or film coated tablets, liquid solutions or suspensions; rectally in the form of suppositories; parenterally, e.g., intramuscularly, or through intravenous and/or intrathecal and/or intraspinal injection or infusion.

In the method of the subject invention, for the administration of bortezomib, the course of therapy generally employed is from about 50 mg/m² to 100 mg/m² every three weeks or from 30 mg/m² weekly.

The anti-neoplastic therapy of the present invention is in particular suitable for treating all forms of cancer including, but not limited to: carcinoma such as bladder, breast, colon, kidney, liver, lung, including small cell lung cancer, esophagus, gall- bladder, ovary, pancreas, stomach, cervix, thyroid, prostate, and skin, including squamous cell carcinoma; haematopoietic tumors of lymphoid lineage including leukaemia, acute lymphocytic leukaemia, acute lymphoblastic leukaemia, B-cell lymphoma, T-cell-lymphoma, Hodgkin's lymphoma, non-Hodgkin's lymphoma, mantle cell lymphoma, hairy cell lymphoma and Burkett's lymphoma; haematopoietic tumors of myeloid lineage, including acute and chronic myelogenous leukemias, myelodysplastic syndrome and promyelocytic leukaemia; multiple myeloma; tumors of mesenchymal origin, including fibrosarcoma and rhabdomyosarcoma; tumors of the central and peripheral nervous system, including astrocytoma neuroblastoma, glioma and schwannomas; other tumors, including melanoma, seminoma, teratocarcinoma, osteosarcoma, xeroderma pigmentosum, keratoxanthoma, thyroid follicular cancer and Kaposi's sarcoma.

As stated above, the effect of the combination of the invention is significantly increased without a parallel increased toxicity. In other words, the combined therapy of the present invention enhances the antitumoral effects of the component (a) and/or of component (b) of the combination of the invention and thus yields the most effective and less toxic treatment for tumors.

Pharmaceutical compositions according to the invention are useful in anticancer therapy.

The present invention further provides a commercial package comprising, in a suitable container means, (a) a compound of formula (A) as defined above, and (b) bortezomib, wherein the active ingredients are present in each case in free form or in the form of a pharmaceutically acceptable salt or any hydrate thereof, together with instructions for simultaneous, separate or sequential use thereof.

In a package according to the invention each of component (a) and (b) are present within a single container means or within distinct container means.

Another embodiment of the present invention is a commercial package comprising a pharmaceutical composition or product as described above.

Due to the key role of the aurora kinase proteins in the regulation of cellular mitosis and proliferation, the combination of the present invention is also useful in the treatment of a variety of cell proliferative disorders such as, for example, benign prostate hyperplasia, familial adenomatosis, polyposis, neurofibromatosis, psoriasis, vascular smooth cell proliferation associated with atherosclerosis, pulmonary fibrosis, arthritis, glomerulonephritis and post-surgical stenosis and restenosis.

The combination of this invention, as modulators of apoptosis, may also be useful in the treatment of cancer, viral infections, prevention of AIDS development in HIV-infected individuals, autoimmune diseases and neurodegenerative disorders. The activities of the combination of the present invention are shown for instance by the following *in vitro* and *in vivo* tests, which are intended to illustrate the present invention.

### Example 1: Materials and methods for in vitro cytotoxic activity testing

Exponentially growing human colon carcinoma (HCT-116) cell lines were seeded and incubated at 37°C in a humidified 5% CO₂ atmosphere. Drugs were added to the experimental culture, and incubations were carried out at 37° C for 72 hours in the dark. Scalar doses of Compound 1 of formula (A) as defined above and antineoplastic agent were added to the medium 24 hours after seeding. Sequential administration schedule was tested (Compound 1 administered 24 hours after the other agent). Drug solutions were prepared immediately before use. At the end of treatment, cell proliferation was determined by an intracellular adenosine triphosphate monitoring system (CellTiterGlo-Promega) using the Envision (PerkinElmer) as reader. Inhibitory activity was evaluated comparing treated versus control data using Assay Explorer (MDL) program. The dose inhibiting 50% of cell growth was calculated using sigmoidal interpolation curve. Combination indices (C.I.) were calculated using a computer program for multiple drug effect analysis based on the equation of Chou-Talalay (Adv Enzyme Regul 1984; 22:27-55) for mutually nonexclusive drugs, where a C.I. <1 indicates a more than additive effect (C.I. >3 indicates strong antagonism; 1.3<C.I.<3, antagonism; 0.8<C.I.<1.3, additivity; 0.3<C.I. < 0.8, synergism; C.I.<0.3, strong synergism).

### Example 2: In vitro cytotoxic activity of the combination with flavopiridol

The results shown in Table 1 indicate that on human colon carcinoma HCT-116 cell line the administration of Compound 1 in combination with flavopiridol resulted in a synergistic antitumor effect.

**Table 1**

| Cell line | Drug | | Schedule | C.I. | Effect of combination |
|---|---|---|---|---|---|
| | Compound 1 µM | Flavopiridol µM | | | |
| HCT-116 | 0.5 | 0.62 | Sequential | 0.63 | Synergism |
| | 0.25 | 0.62 | | 0.63 | Synergism |

### Example 3: In vivo antitumor efficacy of the combination with the monoclonal antibody bevacizumab

Balb, Nu\Nu male mice, from Harlan (Italy), were maintained in cages with paper filter cover, food and bedding sterilized and water acidified. 2.5 x10⁶ DU145 prostate carcinoma cells (from the American Type Culture Collection) were injected subcutaneously in athymic mice. This tumor model was selected because it was previously demonstrated that Bevacizumab inhibits angiogenesis and growth of the model in vivo (see for reference The Prostate 36: 1-10, 1998). The treatment started at day 9 after cell injection, when tumors were palpable with a tumor weight mean for all the groups of 0.15 g. Bevacizumab was prepared immediately before treatment, while Compound 1 was prepared daily, on the basis of known stability of the compound.

Compound 1 was administrated by intraperitoneal route in a volume of 10 ml/kg at the dose of 15 mg\kg twice a day (BID) for 9 days from day 9 to day 17. Bevacizumab was administered intraperitoneally in a volume of 10 ml/kg at the dose of 20 mg/kg on days 9, 13, 17 from the days of tumor cells injection. When combined, Compound 1 was administered at days 9, 10, 11, 12, 13, 14,15, 16, 17, 18 and 19, bevacizumab was administered at days 9, 13 and 17 immediately after Compound 1 administration. Tumor growth and body weight were measured every 3 days. Tumor growth was assessed by caliper. The two diameters were recorded and the tumor weight was calculated according the following formula: length (mm) x width²/2 .The effect of the antitumor treatment was evaluated as the delay in the onset of an exponential growth of the tumor (see for references, Anticancer drugs 7:437-60, 1996). This delay (T-C value) was defined as the difference of time (in days) required for the treatment group (T) and the control group(C) tumors to reach a predetermined size (1g). Toxicity was evaluated on the basis of body weight reduction. The results are reported in Table 2 below. Compound 1 combined with bevacizumab produced a clear additive/synergistic effect: the T-C observed when Compound 1 was combined with bevacizumab was superior to the expected by the simple addition of T-C obtained by the single treatments. No toxicity was observed in any of the treatment group.

**Table 2**

| Treatment | Time to reach 1g (days) | T-C (days) | Toxicity |
|---|---|---|---|
| Compound 1 15 mg/kg* | 23,9 | 8.5 | 0/8 |
| Bevacizumab 20 mg/kg** | 18.5 | 3.1 | 0/8 |
| Bevacizumab 20 mg/kg + Compound 1 15 mg/kg*** | 28.2 | 12.8 | 0/8 |

| | | | |
|---|---|---|---|
| *Treatments made intraperitoneally twice at days 9,10,11,12,13,14,15,16, 17, 18 and 19 **Treatments made intraperitoneally at days 9, 13 and 17 *** Days 9, 13 and 17 bevacizumab treatments; days 9, 10, 11, 12, 13, 14, 15, 16, 17, 18 and 19 Compound 1 treatments | | | |

### Example 4: In vivo antitumor efficacy of the combination with bortezomib

SCID male mice, from Harlan (Italy), were maintained in cages with paper filter cover, food and bedding sterilized and water acidified. 5x10⁶ HL-60 human acute myeloid leukaemia cells (from the American Type Culture Collection) were injected subcutaneously in SCID mice. This tumor model was selected because it was previously demonstrated that Compound 1 inhibits growth of the model in vivo and also on the basis of use of this model as representative of haematological malignancy.

The treatment started 12 days later tumor cells injection when tumors were palpable. Bortezomib was prepared immediately before treatment, Compound 1 was prepared daily, on the basis of known stability of the compound.

Compound 1 was administered by intraperitoneal route in a volume of 10 ml/kg at the doses of 15 mg\kg twice a day (BID) for 12 days (days 12 to 23). Bortezomib was administered by intravenous route in a volume of 10 ml/kg at the dose of 0.5 mg/kg on days 12, 16, 20 and 24 from the days of cell injection, and at the dose of 1 mg/kg on days 12, 16, 20 and 24 from the days of cell injection. When combined, Compound 1 was administered in the interval between the bortezomib treatments at days 13, 14, 15, 17, 18, 19, 21, 22, 23, 25, 26 and 27. Tumor growth and body weight were measured every 3 days. Tumor growth was assessed by caliper. The two diameters were recorded and the tumor weight was calculated according the following formula:
length (mm) x width² /2 .The effect of the antitumor treatment was evaluated as the delay in the onset of an exponential growth of the tumor (see for references, Anticancer drugs 7:437-60, 1996). This delay (T-C value) was defined as the difference of time (in days) required for the treatment group (T) and the control group (C) tumors to reach a predetermined size (1g). Toxicity was evaluated on the basis of body weigh reduction. The results are reported in Table 3 below. Compound 1 combined with 0.5 mg/kg of bortezomib produced a clear additive/ synergistic effect: the T-C observed when Compound 1 was combined with 0.5 mg/kg of bortezomib was superior to the expected by the simple addition of T-C obtained by the single treatments. No toxicity was observed in the treatment groups. Compound 1 combined with 1 mg/kg of bortezomib produced a strong synergistic effect: the T-C observed when Compound 1 was combined with 1 mg/kg of bortezomib (35.7) was clearly superior to the expected by the simple addition of T-C obtained by the single treatments (17.3). Some signs of toxicity were observed in the treatment groups.

**Table 3**

| Treatment | Time to reach 1 g (days) | T-C (days) | Toxicity |
|---|---|---|---|
| Compound 1 15 mg/kg* | 24.7 | 9.1 | 0/7 |
| Bortezomib 0.5 mg/kg** | 19 | 3.4 | 0/7 |
| Bortezomib 1 mg/kg** | 23.8 | 8.2 | 0/7 |
| Bortezomib 0.5 mg/kg + | 29.2 | 13.5 | 0/7 |
| Compound 1 15 mg/kg*** | | | |
| Bortezomib 1 mg/kg + | 51.4 | 35.7 | 3/7 |
| Compound 1 15 mg/kg*** | | | |

| | | | |
|---|---|---|---|
| *Treatments made intraperitoneally twice at days 12,13,14,15,16,17,18, 19, 20, 21, 22,23. **Treatments made by intravenous route at days 12,16,20,24 *** Days 12,16, 20 24 bortezomib treatments, 13, 14, 15,17,18, 19, 21, 22, 23, 25, 26 and 27 Compound 1 treatments. | | | |

## Claims

1. A therapeutic combination comprising (a) Compound 1 of formula (A): 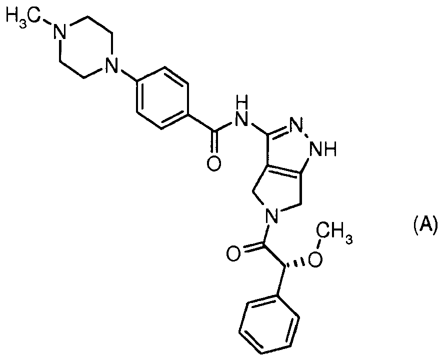 and (b) bortezomib, wherein active ingredients of the combination are present in each case in free form or in the form of a pharmaceutically acceptable salt or any hydrate thereof.

2. The combination according to claim 1 which is a combined preparation for simultaneous, separate or sequential use.

3. The combination according to any one of claims 1 to 2 for use in a method of treating or delaying the progression of a proliferative disorder, wherein the said method comprises a simultaneous, sequential or separate administration to a patient in need thereof of a therapeutically effective amount of the said combination.

4. A pharmaceutical composition comprising a combination according to any one of claims 1 to 2 admixed with a pharmaceutically acceptable carrier, diluent or excipient.

5. A combined preparation comprising compound 1 of formula (A) according to claim 1 and bortezomib for use in lowering the side effects caused by antineoplastic therapy with an antineoplastic agent in mammals, including humans, in need thereof, wherein the said use comprises administering to said mammal the said preparation in amounts effective to produce a synergistic antineoplastic effect.

6. A commercial package comprising, in a suitable container means, (a) compound 1 of formula (A) as defined in claim 1, and (b) bortezomib wherein the active ingredients are present in each case in free form or in the form of a pharmaceutically acceptable salt or any hydrate thereof, together with instructions for simultaneous, separate or sequential use thereof.

## Patentansprüche

1. Therapeutische Kombination umfassend (a) Verbindung 1 mit der Formel (A): 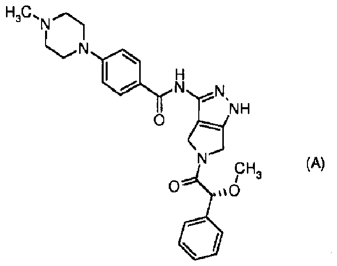 und (b) Bortezomib, wobei aktive Inhaltsstoffe der Kombination in jedem Fall in freier Form oder in der Form eines pharmazeutisch zulässigen Salzes oder eines Hydrats davon vorhanden sind.

2. Kombination nach Anspruch 1, bei der es sich um eine kombinierte Zubereitung für die simultane, separate oder aufeinander folgende Verwendung handelt.

3. Kombination nach einem der Ansprüche 1 bis 2 für die Verwendung in einem Verfahren des Behandelns oder Verzögerns des Fortschreitens einer proliferativen Erkrankung, wobei das Verfahren eine simultane, separate oder aufeinander folgende Verabreichung einer therapeutisch wirksamen Menge der Kombination an einen Patienten, der diese benötigt, umfasst.

4. Pharmazeutische Zusammensetzung, umfassend eine Kombination nach einem der Ansprüche 1 bis 2 in Beimischung mit einem pharmazeutisch zulässigen Träger, Verdünnungsmittel oder Exzipienten.

5. Kombinierte Zubereitung, umfassend Verbindung 1 mit der Formel (A) nach Anspruch 1 und Bortezomib, für die Verwendung beim Verringern der Nebenwirkungen, die durch eine antineoplastische Therapie mit einem antineoplastischen Mittel verursacht werden, in Säugetieren, einschließlich Menschen, die diese benötigen, wobei die Verwendung das Verabreichen der Zubereitung in Mengen, die zum Bewirken eines synergistischen antineoplastischen Effekts wirksam sind, an das Säugetier umfasst.

6. Kommerzielle Packung, umfassend (a) Verbindung 1 mit der Formel (A), wie in Anspruch 1 definiert, und (b) Bortezomib, wobei die aktiven Inhaltsstoffe in jedem Fall in freier Form oder in der Form eines pharmazeutisch zulässigen Salzes oder eines Hydrats davon vorhanden sind, zusammen mit Anweisungen für deren simultane, separate oder aufeinander folgende Verwendung in einem geeigneten Behältermittel.

## Revendications

1. Combinaison thérapeutique comprenant (a) le composé 1 de formule (A) : 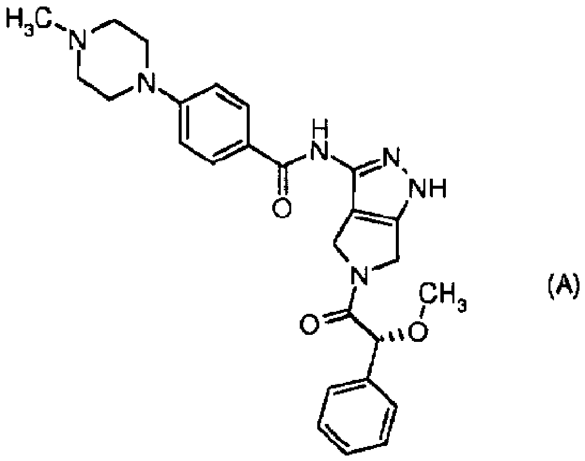 et (b) du bortezomib, dans laquelle les principes actifs de la combinaison sont présents dans chaque cas sous la forme libre ou sous la forme d'un sel pharmaceutiquement acceptable ou de tout hydrate de ceux-ci.

2. Combinaison selon la revendication 1, qui est une préparation combinée destinée à une utilisation simultanée, séparée ou séquentielle.

3. Combinaison selon l'une quelconque des revendications 1 à 2, destinée à être utilisée dans un procédé de traitement ou de ralentissement de la progression d'un trouble de la prolifération, ledit procédé comprenant une administration simultanée, séquentielle ou séparée à un patient le nécessitant d'une quantité thérapeutiquement efficace de ladite combinaison.

4. Composition pharmaceutique comprenant une combinaison selon l'une quelconque des revendications 1 à 2 mélangée avec un vecteur, un diluant ou un excipient pharmaceutiquement acceptables.

5. Préparation combinée comprenant le composé 1 de formule (A) selon la revendication 1 et dubortezomib, destinée à être utilisée pour réduire les effets secondaires causés par une thérapie antinéoplasique avec un agent antinéoplasique chez les mammifères, y compris les humains, le nécessitant, ladite utilisation comprenant l'administration au dit mammifère de ladite préparation en quantités efficaces pour produire un effet antinéoplasique synergique.

6. Emballage commercial comprenant, dans un moyen formant récipient adapté, (a) le composé 1 de formule (A) selon la revendication 1, et (b) le bortezomib, dans lequel les principes actifs sont présents dans chaque cas sous la forme libre ou sous la forme d'un sel pharmaceutiquement acceptable ou de tout hydrate de ceux-ci, conjointement à une notice relative à son utilisation simultanée, séparée ou séquentielle.
